# EUROPEAN PATENT APPLICATION

(11) **EP 4 005 616 A1**
(43) Date of publication of application: **01.06.2022**
(21) Application number: 20844583.3
(22) Date of filing: 22.07.2020
(51) Int. Cl.: A61M 5/142, A61M 5/168, A61M 5/31

(54) **DRUG SOLUTION INJECTION CONTROLLER**

(30) Priority: 22.07.2019 JP 2019134453
(71) Applicant: Nipro Corporation, Osaka-shi, Osaka 531-8510 (JP)
(72) Inventor: SAKAMOTO, Shingo, Osaka-shi, Osaka 531-8510 (JP)
(74) Representative: Slingsby Partners LLP
(86) International application number: PCT/JP2020/028583
(87) International publication number: WO 2021/015281

(57) **Abstract**

A drug solution injection controller 10 includes a sub-reservoir 24 connected via a sub-line 26 to a main line 14 for performing continuous administration of a drug solution such that rapid administration of the drug solution from the sub-reservoir 24 by self-operation is enabled, and comprises: a priming flow path 46 configured to perform priming on at least one of the main line 14 and the sub-line 26; a closing mechanism 48 configured to close the priming flow path 46; and a connector holding part 84 holding a terminal connector 18 connected to the main line 14. Holding of the terminal connector 18 by the connector holding part 84 is allowed to be released due to operation of an operation switch 50 configured to activate the closing mechanism 48.

## Description

### TECHNICAL FIELD

The present invention relates to a drug solution injection controller that enables rapid injection of a drug solution by self-operation in a drug solution administration device for continuous administration of the drug solution.

### BACKGROUND ART

Conventionally, a drug solution administration device that continuously administers a drug solution has been known and is used, for example, when continuously administering painkillers, anesthetics, etc. into the body little by little. Further, the drug solution administration device may include, for example, a drug solution injection controller that enables rapid administration of the drug solution by self-operation, as described in International Publication No. WO 2017/098685 A1 (Patent Document 1). The drug solution injection controller is provided with a sub-reservoir for storing the drug solution. For example, by the patient performing a self-operation such as pushing a pressing operation member, the drug solution stored in the sub-reservoir of the drug solution injection controller is rapidly administered into the patient's body.

Meanwhile, in order to prevent air bubbles from entering blood vessels and the like, the drug solution administration device is subjected to priming in which the entire administration line of the drug solution is filled with a priming liquid before use.

### BACKGROUND ART DOCUMENT

### PATENT DOCUMENT

Patent Document 1: WO 2017/098685 A1

### SUMMARY OF THE INVENTION

### PROBLEM THE INVENTION ATTEMPTS TO SOLVE

However, since the drug solution administration device is a device that continuously administers a small amount of the drug solution, there is a problem that it takes time for the priming solution to fill the entire administration line of the drug solution.

Therefore, for example, it is conceivable to reduce the time required for priming by providing the portion of the drug solution administration line that limits the flow rate with a priming flow path having a larger flow rate in parallel.

However, in the case where a closing mechanism for closing the priming flow path is provided, the order and timing of activating the closing mechanism of the priming flow path are important. That is, for example, if a terminal connector of the drug solution administration device is connected to the indwelling needle or the like on the patient side without closing the priming flow path, there is a risk that an excessive drug solution may be administered into the patient's body through the priming flow path. Another example is that the closing mechanism may be activated before priming, posing a risk of the priming flow path becoming unavailable during priming.

It is an object of the present invention to provide a drug solution injection controller with a novel structure capable of reducing a situation in which an operation switch for activating a closing mechanism of a priming flow path is not operated correctly at an appropriate timing.

### MEANS FOR SOLVING THE PROBLEM

Hereinafter, preferred embodiments for grasping the present invention will be described. However, each preferred embodiment described below is exemplary and can be appropriately combined with each other. Besides, a plurality of elements described in each preferred embodiment can be recognized and adopted as independently as possible, or can also be appropriately combined with any element described in other preferred embodiments. By so doing, in the present invention, various other preferred embodiments can be realized without being limited to those described below.

A first preferred embodiment provides a drug solution injection controller including a sub-reservoir connected via a sub-line to a main line for performing continuous administration of a drug solution such that rapid administration of the drug solution from the sub-reservoir by self-operation is enabled, the drug solution injection controller comprising: a priming flow path configured to perform priming on at least one of the main line and the sub-line; a closing mechanism configured to close the priming flow path; and a connector holding part holding a terminal connector connected to the main line, wherein holding of the terminal connector by the connector holding part is allowed to be released due to operation of an operation switch configured to activate the closing mechanism.

According to the drug solution injection controller of the present preferred embodiment, holding of the terminal connector by the connector holding part is allowed to be released due to operation of the operation switch. Thus, it is possible to prevent the terminal connector from being connected to the indwelling needle or the like, which is the flow path on the patient side, before the operation switch is operated. It is possible to prevent it from being connected to a certain indwelling needle or the like. Therefore, the administration of the drug solution to the patient side will not be accidentally started before the priming flow path is closed, and an overdose of the drug solution through the priming flow path is avoided.

A second preferred embodiment provides the drug solution injection controller according to the first preferred embodiment, wherein the connector holding part is provided to a holding member separate from the operation switch, and holding of the terminal connector by the connector holding part is allowed to be released by operation of the holding member due to operation of the operation switch.

According to the drug solution injection controller of the present preferred embodiment, for example, by detaching the holding member from the operation switch or making the holding member with a soft material and deforming the holding member, it is possible to allow the holding of the terminal connector by the connector holding part to be released.

A third preferred embodiment provides a drug solution injection controller including a sub-reservoir connected via a sub-line to a main line for performing continuous administration of a drug solution such that rapid administration of the drug solution from the sub-reservoir by self-operation is enabled, the drug solution injection controller comprising: a priming flow path configured to perform priming on at least one of the main line and the sub-line; a closing mechanism configured to close the priming flow path; and an action preventing mechanism preventing operation of an operation switch configured to activate the closing mechanism, wherein activation of the closing mechanism by operation of the operation switch is allowed due to release of the action preventing mechanism.

According to the drug solution injection controller of the present preferred embodiment, closing of the priming flow path by the operation of the operation switch is prevented by the action preventing mechanism. With this configuration, for example, it is possible to prevent the priming flow path from being closed by erroneously operating the operation switch before the priming is started. By releasing the action preventing mechanism after the priming is completed, the priming flow path can be closed by the closing mechanism.

A fourth preferred embodiment provides the drug solution injection controller according to the third preferred embodiment, wherein the action preventing mechanism is constituted by an action preventing member separate from the operation switch being attached to the operation switch.

According to the drug solution injection controller of the present preferred embodiment, it is possible to avoid misoperation of the operation switch before the completion of priming, and it is also possible to prevent the use from forgetting to operate the operation switch after the completion of priming.

A fifth preferred embodiment provides a drug solution injection controller including a sub-reservoir connected via a sub-line to a main line for performing continuous administration of a drug solution such that rapid administration of the drug solution from the sub-reservoir by self-operation is enabled, the drug solution injection controller comprising: a priming flow path configured to perform priming on at least one of the main line and the sub-line; a closing mechanism configured to close the priming flow path; a display part having an information display function for priming-related information; and a display obstruction mechanism configured to obstruct the information display function of the display part due to operation of an operation switch configured to activate the closing mechanism.

According to the drug solution injection controller of the present preferred embodiment, it is possible to display priming-related information on the display part. The priming-related information includes, for example, alphanumeric characters such as "Do not operate before completion of priming" and "Remember to operate after completion of priming", as well as a warning statement indicated by graphic symbols, colors, numbers representing the order, etc., and an operation method, or the like. With this configuration, the health care worker can obtain information related to the operation of the operation switch when operating the operation switch.

When the operation of the operation switch is completed, the priming-related information displayed on the display part is not only unnecessary, but may also confuse the patient or the like. Therefore, for example, by providing the display obstruction mechanism such that the display part can be removed due to operation of the operation switch, the information display function by the display part is obstructed after the operation of the operation switch is completed. By so doing, after the operation of the operation switch is completed, it is possible to prevent confusion of the patient or the like due to the display of unnecessary information.

A sixth preferred embodiment provides the drug solution injection controller according to the fifth preferred embodiment, wherein the display part is provided to a display member separate from the operation switch and attached to the operation switch, and the display part is allowed to be removed by the display member being detached from the operation switch due to operation of the operation switch.

According to the drug solution injection controller of the present preferred embodiment, the display obstruction mechanism is constituted by the separate display member attached to the operation switch being detached from the operation switch so as to be removed from the drug solution injection controller. With this configuration, the obstruction of the information display function of the display part by the display obstruction mechanism can be easily and surely realized. For example, by providing a step of detaching the display member from the operation switch in the operation process of the operation switch, the display part can also be removed due to the operation of the operation switch to eliminate the information display by the display part.

A seventh preferred embodiment provides the drug solution injection controller according to the first or second preferred embodiment, wherein the holding member is detached from the operation switch due to operation of the operation switch, and holding of the terminal connector by the connector holding part is allowed to be released.

According to the drug solution injection controller of the present preferred embodiment, before the operation of the operation switch is completed, the terminal connector is prevented from being connected to the patient side, and after the operation of the operation switch is completed, holding of the terminal connector is allowed to be released by the holding member being detached, so as to allow the terminal connector to be connected to the patient side.

An eighth preferred embodiment provides the drug solution injection controller according to the second or seventh preferred embodiment, wherein the action preventing mechanism preventing operation of the operation switch configured to activate the closing mechanism includes the holding member, and the action preventing mechanism is configured to be released by operation of the holding member due to operation of the operation switch.

According to the drug solution injection controller of the present preferred embodiment, the action preventing mechanism prevents the priming flow path from being closed due to the operation of the operation switch. With this configuration, for example, it is possible to prevent the operation switch from being accidentally operated before the start of priming or the completion of priming to close the priming flow path.

The action preventing mechanism is released by operation such as deformation of the holding member or detachment of the holding member from the operation switch due to operation of the operation switch, and the priming flow path is allowed to be closed by the operation of the operation switch. Then, for example, by allowing the holding of the terminal connector by the connector holding part to be released due to operation of the operation switch that completes the closing of the priming flow path, the terminal connector will not be connected to the patient side before the priming flow path is closed.

A ninth preferred embodiment provides the drug solution injection controller according to any one of the second, seventh, and eighth preferred embodiments, wherein a display part having an information display function for priming-related information is provided to the holding member.

According to the drug solution injection controller of the present preferred embodiment, the display part is provided to the holding member separate from the operation switch. With this configuration, for example, in the state where operation of the operation switch is completed, it is also possible to obstruct the information display function of the display part by deforming or detaching the holding member. By so doing, when operating the operation switch, it is possible to let the user confirm the operation method or to call attention by the display on the display part. Besides, in the state where operation of the operation switch is completed, it is possible to prevent the patient or the like from being confused due to the display of unnecessary information.

A tenth preferred embodiment provides the drug solution injection controller according to the fourth preferred embodiment, wherein a display part having an information display function for priming-related information is provided to the action preventing member.

According to the drug solution injection controller of the present preferred embodiment, the display part is provided to the action preventing member separate from the operation switch. With this configuration, for example, in the state where operation of the operation switch is completed, it is also possible to obstruct the information display function of the display part by deforming or detaching the action preventing member. By so doing, when operating the operation switch, it is possible to let the user confirm the operation method or to call attention by the display on the display part. Besides, in the state where operation of the operation switch is completed, it is possible to prevent the patient or the like from being confused due to the display of unnecessary information.

An eleventh preferred embodiment provides a drug solution injection controller including a sub-reservoir connected via a sub-line to a main line for performing continuous administration of a drug solution such that rapid administration of the drug solution from the sub-reservoir by self-operation is enabled, the drug solution injection controller comprising: a priming flow path configured to perform priming on at least one of the main line and the sub-line; a closing mechanism configured to close the priming flow path; and a display part having an information display function for priming-related information including operation information of the closing mechanism.

According to the drug solution injection controller of the present preferred embodiment, it is possible to display priming-related information on the display part. The priming-related information includes, for example, alphanumeric characters such as "Do not operate before completion of priming" and "Remember to operate after completion of priming", as well as a warning statement indicated by graphic symbols, colors, numbers representing the order, etc., and an operation method, or the like. With this configuration, the health care worker can obtain the priming-related information such as the operation information of the closing mechanism by operation of the operation switch when operating the operation switch.

### EFFECT OF THE INVENTION

According to the present invention, it is possible to reduce the situation in which the operation switch for activating the closing mechanism of the priming flow path is not operated correctly at an appropriate timing.

### BRIEF DESCRIPTION OF THE DRAWINGS

FIG. 1 is a top plan view of a drug solution administration device including a drug solution injection controller as a first practical embodiment of the present invention.
FIG. 2 is a perspective view of the drug solution injection controller constituting the drug solution administration device shown in FIG. 1.
FIG. 3 is a perspective view showing the drug solution injection controller of FIG. 2 at another angle.
FIG. 4 is a top plan view of the drug solution injection controller shown in FIG. 2.
FIG. 5 is a cross sectional view of the drug solution injection controller shown in FIG. 2.
FIG. 6 is a perspective view of an operation switch constituting the drug solution injection controller shown in FIG. 2.
FIG. 7 is a perspective view of a tab member constituting the drug solution injection controller shown in FIG. 2.
FIG. 8 is a perspective cross sectional view showing the drug solution injection controller shown in FIG. 2 cut along line 8-8 of FIG. 4.
FIG. 9 is a perspective view of the operation switch and the tab member constituting the drug solution injection controller shown in FIG. 2.
FIG. 10 is a view corresponding to the perspective cross section of the drug solution injection controller shown in FIG. 8, which shows the state where the tab member is pulled out halfway.
FIG. 11 is a view corresponding to the perspective cross section of the drug solution injection controller shown in FIG. 8, which shows the state where the operation switch is pushed in.
FIG. 12 is a perspective view of the operation switch and the tab member constituting the drug solution injection controller shown in FIG. 11.
FIG. 13 is a view corresponding to the perspective cross section of the drug solution injection controller shown in FIG. 8, which shows the state where the tab member is detached from the operation switch.
FIG. 14 is a perspective view of a drug solution injection controller as a second practical embodiment of the present invention.
FIG. 15 is a perspective view showing the drug solution injection controller shown in FIG. 14 at another angle.

### EMBODIMENTS FOR CARRYING OUT THE INVENTION

Hereinafter, practical embodiments of the present invention will be described with reference to the drawings.

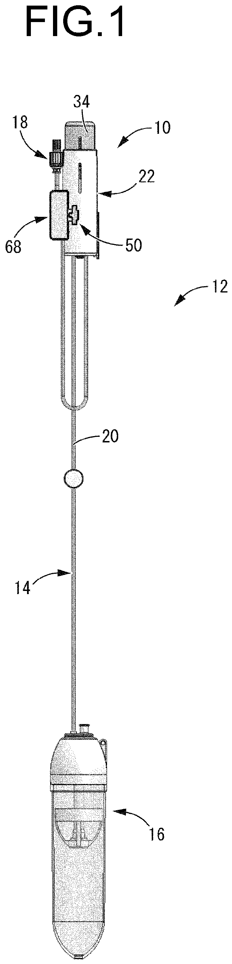

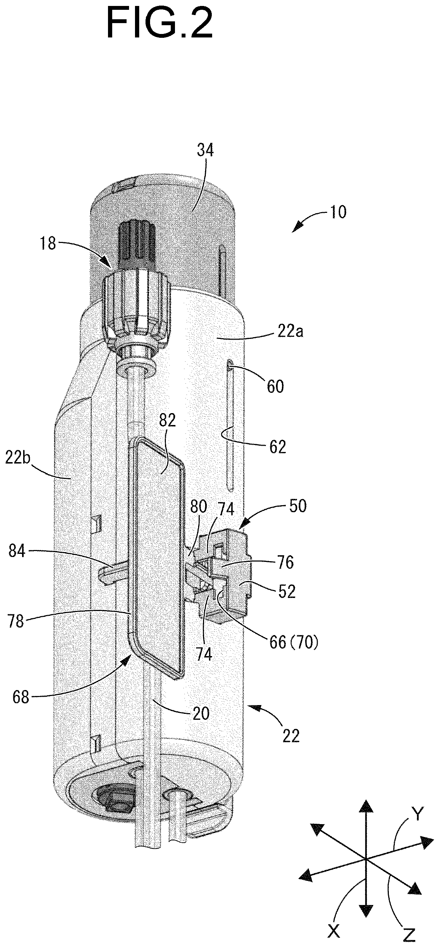

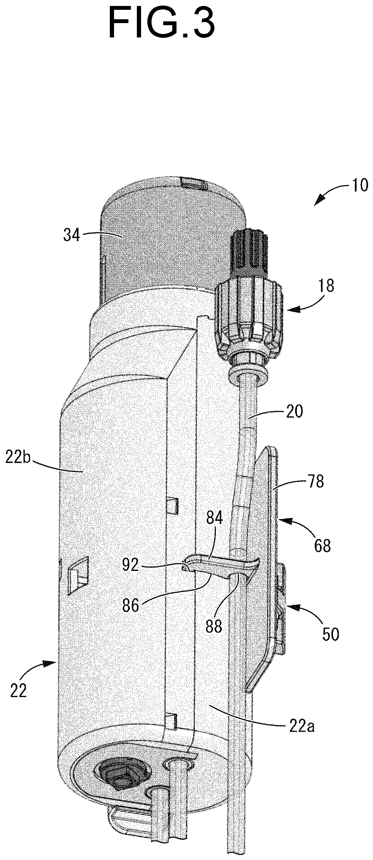

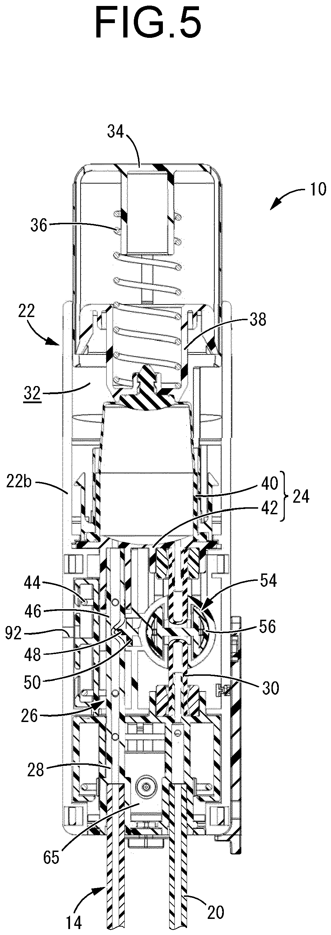

FIG. 1 depicts a drug solution administration device 12 including a drug solution injection controller 10 according to a first practical embodiment of the present invention. The drug solution administration device 12 has a structure in which a main reservoir 16 that is configured to deliver the drug solution stored inside to a main line 14, and a terminal connector 18 that is configured to be connected to an indwelling needle (not shown) percutaneously stuck into a patient's blood vessel or the like are connected by the main line 14. Moreover, the drug solution injection controller 10 shown in FIGS. 2 to 5 is connected to an external flow path 20 constituting the main line 14. In the following description, the distal end side of the drug solution injection controller 10 is the lower side in FIG. 5, and the proximal end side of the drug solution injection controller 10 is the upper side in FIG. 5.

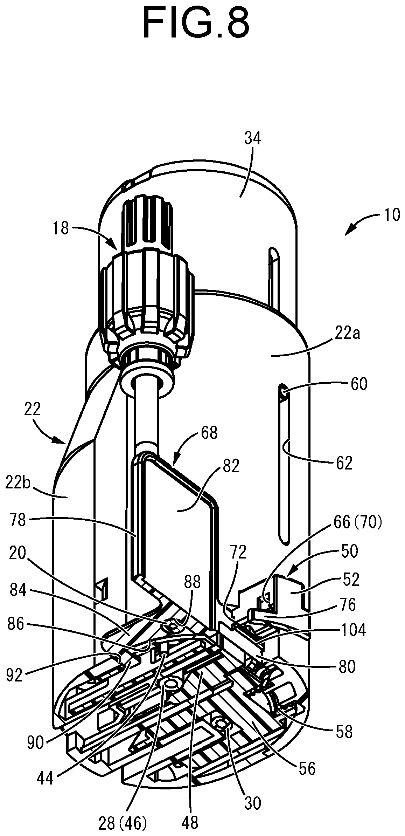

As shown in FIG. 5, the drug solution injection controller 10 has a structure in which a sub-reservoir 24 is housed in a housing 22, and includes a sub-line 26 that branches from the main line 14 and connects the sub-reservoir 24 to the main line 14. The sub-line 26 includes a part of an inflow line 28 on the upstream side of the sub-reservoir 24 and a part of an outflow line 30 on the downstream side of the sub-reservoir 24. The ends of the inflow line 28 and the outflow line 30 are each exposed from the housing 22 to the distal end side, and the end of the inflow line 28 constitutes an inlet port connected to the external flow path 20 on the upstream side, while the end of the outflow line 30 constitutes an outlet port connected to the external flow path 20 on the downstream side. In FIGS. 2 to 5 depicting the drug solution injection controller 10 and FIGS. 8, 10, 11 and 13 described later, a part of the external flow path 20 and the terminal connector 18 are depicted to facilitate understanding.

The housing 22 has an approximately round tubular shape with a bottom that opens toward the proximal end side, and has a hollow structure including a housing area 32 inside. The housing 22 has a structure in which a first half body 22a and a second half body 22b are combined in the axis-perpendicular direction. Further, a push button 34 is attached to the proximal end portion of the housing 22 so as to be relatively movable in the axial direction (the vertical direction in FIG. 5). The push button 34 is elastically positioned at the proximal end portion of the housing 22 by the elasticity of a coil spring 36 arranged in an inserted state. A plunger 38 is provided on the distal end side of the coil spring 36, and the coil spring 36 is interposed axially between the plunger 38 and the push button 34.

The sub-reservoir 24 is arranged on the distal end side of the plunger 38. The sub-reservoir 24 is constituted by a concave diaphragm part 40 that opens toward the distal end and a base member 42 that covers the opening of the diaphragm part 40. Then, the volume of the sub-reservoir 24 will be changed by the bottom wall part of the diaphragm part 40 being pushed toward the distal end side by the plunger 38. The push button 34, the plunger 38, and the sub-reservoir 24 are arranged side by side in series in the axial direction, which is the longitudinal direction of the housing 22.

The inflow line 28 and the outflow line 30 are connected to the base member 42 of the sub-reservoir 24. The inflow line 28 is branched at a portion constituting the sub-line 26, and a limiting flow path 44 and a priming flow path 46 are provided in parallel. The priming flow path 46 has a flow path cross-sectional area larger than that of the limiting flow path 44, so as to have a larger flow rate.

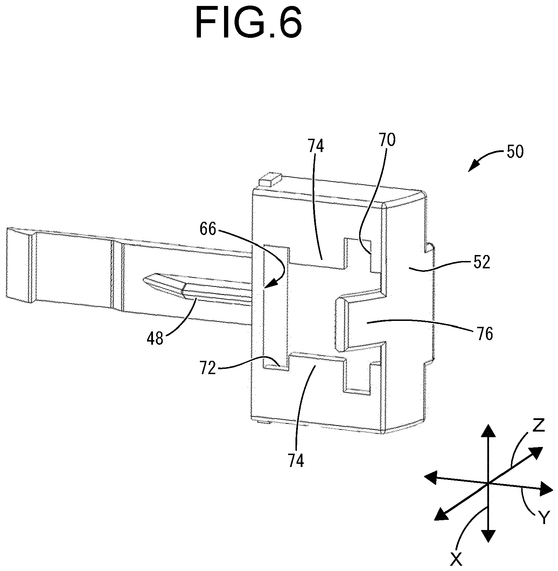

A closing valve 48 serving as a closing mechanism capable of closing the priming flow path 46 is provided on the priming flow path 46. As shown in FIG. 6, the closing valve 48 is integrally formed with an operation switch 50 exposed on the outer peripheral surface of the housing 22. The operation switch 50 is movable relative to the housing 22, and the closing valve 48 moves from the open position to the closed position shown in FIG. 5 by a push-in operation of pushing an operation surface 52 of the operation switch 50 with a finger. Although the operation switch 50 of the present practical embodiment is designed to be pushed in, the operation of the operation switch is not particularly limited, and may be, for example, an operation of pulling the operation switch to the radially outer side of the housing 22, or an operation of sliding the operation switch in the axial direction of the housing 22 or the like, or an operation of rotating the operation switch.

The operation switch 50 is operated after the completion of priming performed before the continuous administration of the drug solution by the drug solution administration device 12 is started. That is, before the completion of priming, the closing valve 48 is in the open position and the priming flow path 46 is in an open state, so that the sub-line 26 and the sub-reservoir 24 are quickly filled with the priming liquid through the priming flow path 46. Preferably, the terminal connector 18 provided at the terminal of the external flow path 20 is provided with a filter that allows the passage of air and blocks the passage of the priming liquid, thereby preventing the outflow of the priming liquid at the downstream end. After the priming is completed, the operation switch 50 is pushed in to move the closing valve 48 to the closed position, and the priming flow path 46 is in a closed state. By so doing, the flow rate of the sub-line 26 is limited by the limiting flow path 44, and the time required for supplying the drug solution to the sub-reservoir 24 after the rapid administration described later is appropriately set. The drug solution injection controller 10 depicted in FIG. 5 is illustrated in a state where the priming is completed, the operation switch 50 is pushed in, and the priming flow path 46 is closed by the closing valve 48.

Meanwhile, an open-close valve 54 is provided on the outflow line 30. The open-close valve 54 is a valve that switches a portion constituting the sub-line 26 in the outflow line 30 between an open state and a closed state. For example, by a valve body 56 being pressed against the outflow line 30 by a coil spring 58 (see FIG. 8 described later), the outflow line 30 is closed by the open-close valve 54, and by the valve body 56 being moved in a direction away from the outflow line 30, the outflow line 30 is switched to the open state. In the present practical embodiment, by the push button 34 being pushed and moved, the valve body 56 is pushed and moved in the direction away from the outflow line 30 by a main drive piece (not shown) extending from the push button 34.

Then, when the push button 34 is pushed in the axial direction by the patient's self-operation, the plunger 38 is urged toward the distal end side by the elasticity of the coil spring 36, so that the sub-reservoir 24 is compressed and the open-close valve 54 is moved from the closed position to the open position. By so doing, the drug solution stored in the sub-reservoir 24 is rapidly administered to the patient side through the outflow line 30 in the open state, and the dose of the drug solution is temporarily increased. In this way, the drug solution injection controller 10 enables rapid administration of the drug solution by the patient's self-operation in the drug solution administration device 12.

Further, the drug solution is supplied through the inflow line 28 to the sub-reservoir 24 in which the amount of the drug solution stored has decreased due to the execution of rapid administration. The priming flow path 46 of the inflow line 28 is closed by the closing valve 48, and the drug solution is supplied little by little to the sub-reservoir 24 through the limiting flow path 44. Thus, a predetermined amount of time will be required until the filling of the drug solution into the sub-reservoir 24 is completed. This avoids the administration of excessive drug solution due to continuous rapid administration. Preferably, another rapid administration is disabled before a predetermined amount of the drug solution is stored in the sub-reservoir 24.

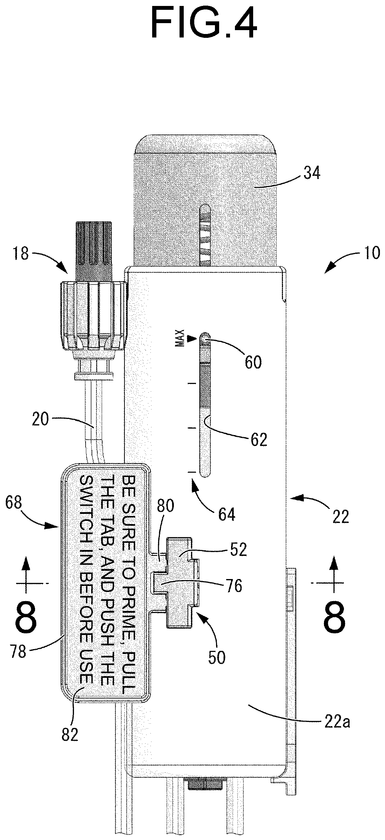

The plunger 38 is provided with a mark part 60 having a shape of protrusion projecting outward in the radial direction, and as shown in FIGS. 2 and 4, the mark part 60 is exposed to the outside through a slit 62 formed in the housing 22. The position of the mark part 60 within the slit 62 changes as the plunger 38 moves. With this configuration, the amount of the drug solution stored in the sub-reservoir 24 can be confirmed by visually checking where the mark part 60 is located in the slit 62. In the present practical embodiment, as shown in FIG. 4, a scale 64 is provided on the lateral side of the slit 62, and the amount of the drug solution in the sub-reservoir 24 according to the position of the mark part 60 can be grasped more accurately.

In the drug solution injection controller 10 of the present practical embodiment, the flow rate of the main line 14 can be switched by a three-way stopcock 65. That is, in the main line 14, the portion housed in the housing 22 is branched into two flow paths provided in parallel, and one flow path is always in an open state to set the lower limit of the flow rate of the main line 14, while the three-way stopcock 65 is provided on the other flow path. With this configuration, the flow rate of the main line 14 can be switched in four steps by switching the three-way stopcock 65, and the drug solution injection controller 10 has an internal flow rate switching mechanism for switching the flow rate of the main line 14. By adopting the drug solution injection controller 10, the drug solution administration device 12 of flow-rate variable type, which is able to change the setting of the amount of the drug solution continuously administered through the main line 14, is constituted. It is desirable that the three-way stopcock 65 can be switched from the outside. Further, the switching of the flow rate of the main line 14 is not necessarily limited to the switching of four steps. The flow rate switching mechanism is not essential in the drug solution injection controller 10, and the flow rate of the main line 14 may be unchangeable.

Meanwhile, the push-in operation of the operation switch 50 is disabled in the initial state of the drug solution injection controller 10. That is, as shown in FIG. 6, the operation switch 50 is provided with a through hole 66. A tab member 68 serving as a holding member is inserted into the through hole 66 and the tab member 68 is attached to the operation switch 50, so that the operation switch 50 is locked to the tab member 68 and the push-in operation is prevented.

The through hole 66 of the operation switch 50 is configured such that in which the width dimension in a first direction X (see FIGS. 2 and 6), which is the axial direction of the housing 22, is made larger in the opposite end portions in a second direction Y (see FIGS. 2 and 6), along which the operation switch 50 is movable with respect to the housing 22, than in the middle portion. In particular, a removal part 70 of the through hole 66, which constitutes the end on the operation surface 52 side of the operation switch 50, is made larger in width dimension in the first direction X than a tab insertion part 72 of the through hole 66, which constitutes the end on the side opposite to the operation surface 52 of the operation switch 50. In the middle portion between the removal part 70 and the tab insertion part 72 of the through hole 66, the width dimension in the first direction X is made small, so as to provide a pair of middle projections 74, 74 projecting inward of the through hole 66 in the first direction X.

At the end of the operation switch 50 on the operation surface 52 side, a regulating protrusion 76 is provided so as to extend from the opening edge of the through hole 66 in the direction of extension of the operation surface 52. The regulating protrusion 76 has a shape that can be inserted into a lightening part 94 of an insertion part 80, which will be described later.

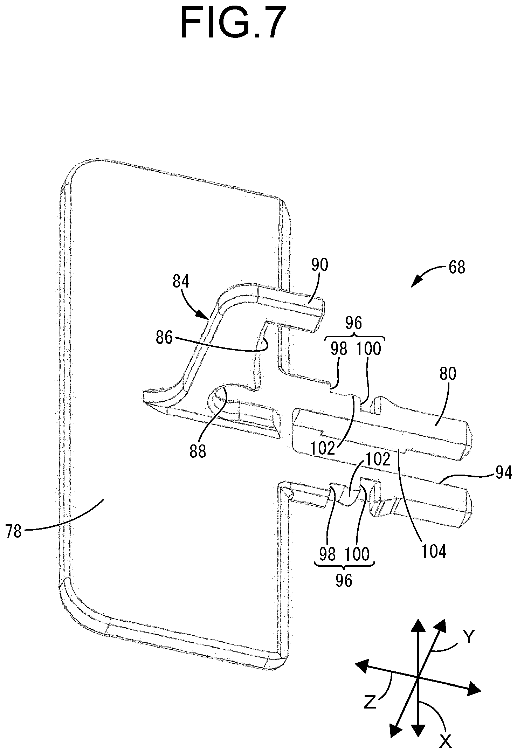

The tab member 68 is formed as an independent member separate from the operation switch 50, and as shown in FIG. 7, includes a plate-shaped display part 78 and the insertion part 80 protruding laterally from the display part 78. The direction of penetration of the through hole 66 in the operation switch 50 is a third direction Z which is approximately orthogonal to the first direction X and the second direction Y. With the operation switch 50 and the tab member 68 attached, the direction of protrusion of the insertion part 80 is the third direction Z.

As shown in FIG. 4, the surface of the display part 78 comprises a display surface 82 on which alphanumeric characters, graphic symbols, colors, numbers representing the order (the procedure), and the like are indicated. On the display surface 82, displayed is priming-related information such as a warning statement regarding the operation of the operation switch 50, for example, "BE SURE TO PRIME, PULL THE TAB, AND PUSH THE SWITCH IN BEFORE USE". In this way, the display part 78 includes an information display function for displaying priming-related information. For the display of the display surface 82 by alphanumeric characters, graphic symbols, colors, numbers representing the order, for example, in addition to the display by coloring such as printing, the display by projections and depressions, sticking of a sticker (a label), and the like are adopted. A display member separate from the operation switch 50 is constituted by the tab member 68 including the display part 78.

As shown in FIG. 7, a connector holding part 84 is provided on the back surface side of the display part 78 of the tab member 68. The connector holding part 84 has a plate shape and protrudes from the back surface of the display part 78. The connector holding part 84 is provided with a concave end 86 that is overlapped with the outer peripheral surface of the housing 22. The concave end 86 has a curved shape along the outer peripheral surface of the first half body 22a.

A tube insertion part 88 having a partially deepened depth is provided at the middle portion of the concave end 86 of the connector holding part 84. The tube insertion part 88 has a concave shape having a width slightly smaller than the outer diameter of the external flow path 20, and by fitting the external flow path 20, the tube insertion part 88 is able to clasp and hold the external flow path 20.

Further, at the end part of the connector holding part 84 on the side far from the display part 78 (the protruding tip part of the connector holding part 84 from the display part 78), a positioning part 90 is provided so as to protrude toward the opening side of the concave end 86 and the tube insertion part 88. The positioning part 90 has a cross-sectional shape corresponding to a positioning hole 92 penetrating the housing 22.

The insertion part 80 has an elongated shape that can be inserted into the through hole 66 of the operation switch 50, and has a bifurcated structure across approximately the entire length by being provided with a lightening part 94 in the widthwise central portion thereof. In the lengthwise middle portion of the insertion part 80, a concave groove 96 opening on the outer surface in the width direction is formed on each side in the width direction, and the width dimension is partially reduced. The concave groove 96 has a shallow bottom part 98 and a deep bottom part 100 having different depths. In the concave groove 96, the side wall on the deep bottom part 100 side, which is the side opposite to the display part 78, protrudes outward in the width direction of the insertion part 80 further than the side wall on the shallow bottom part 98 side, which is the display part 78 side, does. With this configuration, the width dimension of the insertion part 80 is made larger in the portion adjacent on the protruding tip side of the insertion part 80 with respect to the deep bottom part 100 than that of the shallow bottom part 98 of the concave groove 96. In the present practical embodiment, the width dimension of the insertion part 80 gradually increases from the protruding tip side of the insertion part 80 toward the deep bottom part 100 in a tapered shape. A gentle convex part 102 that projects outward in the width direction is provided at a portion of the shallow bottom part 98 that is adjacent to the deep bottom part 100.

A pair of guide ribs 104, 104 protruding in the second direction Y are integrally formed with the insertion part 80. The guide ribs 104 are provided on the opposite sides of the lightening part 94 in the insertion part 80, and extend straightly in the third direction Z, which is the length direction of the insertion part 80.

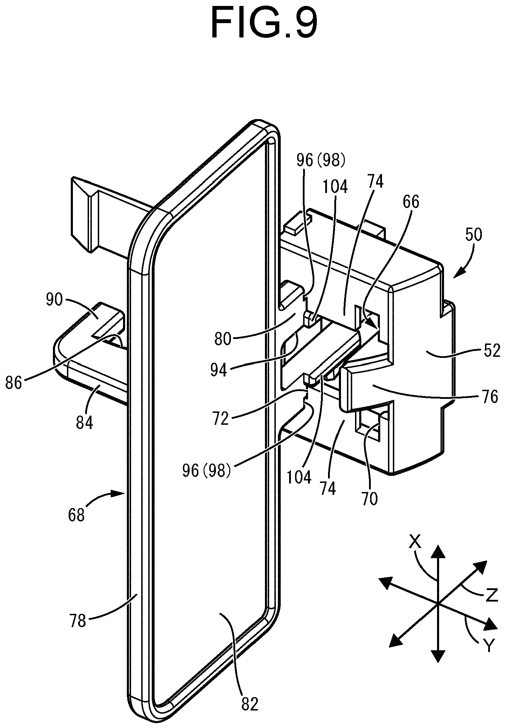

The tab member 68 is attached to the operation switch 50 as shown in FIGS. 2 to 4, 8 and 9. That is, the insertion part 80 of the tab member 68 is inserted through the through hole 66 of the operation switch 50. In the initial state before the operation switch 50 is pushed in, as shown in FIGS. 8 and 9, the insertion part 80 of the tab member 68 is inserted in the tab insertion part 72 of the through hole 66. The side wall inner surface on the shallow bottom part 98 side of each concave groove 96 of the insertion part 80 is overlapped with the opening edge of the tab insertion part 72 of the through hole 66, and the insertion part 80 is positioned with respect to the through hole 66 in the direction of insertion. The pair of guide ribs 104, 104 of the insertion part 80 are inserted between the pair of middle projections 74, 74 of the operation switch 50.

By the insertion part 80 of the tab member 68 being inserted into the through hole 66, the middle projections 74, 74 of the operation switch 50 are overlapped with the bottom wall portions of the shallow bottom parts 98, 98 of the tab member 68 in the second direction Y. With this configuration, the push-in operation of the operation switch 50 with respect to the housing 22 is prevented by the contact locking with the tab member 68. As a result, even if a force in the push-in direction is applied to the operation surface 52 of the operation switch 50 before the completion of priming, the operation switch 50 will not be pushed into the housing 22, and the closing valve 48 will not close. An action preventing member is constituted by the tab member 68 locked to the operation switch 50. The tab member 68 is attached to the operation switch 50, and the operation switch 50 and the tab member 68 are locked to each other, thereby constituting an action preventing mechanism for preventing the closing action of the closing valve 48. In the present practical embodiment, the action preventing mechanism is constituted by direct contact and locking between the operation switch 50 and the tab member 68. However, it would also be possible to constitute the action preventing mechanism by, for example, indirect locking between the operation switch 50 and the tab member 68 via another member.

The external flow path 20 is fitted to the tube insertion part 88 in the vicinity of the end on the terminal connector 18 side. By the connector holding part 84 being attached to the outer peripheral surface of the housing 22 in an overlapped state, the terminal connector 18 is held on the outer peripheral surface of the drug solution injection controller 10 by the connector holding part 84.

That is, by the tab member 68 being attached to the operation switch 50, the concave end 86 of the connector holding part 84 of the tab member 68 is overlapped with the outer peripheral surface of the housing 22. With this arrangement, the circumferentially opened portion of the tube insertion part 88 is closed by the housing 22, so that the external flow path 20 inserted through the tube insertion part 88 will not laterally disengage from the tube insertion part 88. Moreover, since the large-diameter terminal connector 18 is provided at the terminal of the external flow path 20, the external flow path 20 will not slip out of the tube insertion part 88 in the length direction, either. Therefore, in the state where the tab member 68 is attached to the operation switch 50, the terminal connector 18 is held close to the lateral side of the housing 22 without being separated from the drug solution injection controller 10. Also, in the state where the external flow path 20 and the terminal connector 18 are held by the connector holding part 84 of the tab member 68, it is difficult to connect the terminal connector 18 to the indwelling needle or the like on the patient side. In this way, the external flow path 20 is held between the connector holding part 84 of the tab member 68 and the housing 22 so as not to move away from the lateral side of the housing 22, thereby constituting connection preventing mechanism of the present practical embodiment.

The connector holding part 84 is positioned with respect to the housing 22 by the positioning part 90 being inserted into the positioning hole 92 of the housing 22, and the connector holding part 84 is made difficult to separate from the outer peripheral surface of the housing 22. However, the configuration in which the positioning part 90 of the connector holding part 84 is inserted into the positioning hole 92 of the housing 22 is not essential.

The display part 78 of the tab member 68 attached to the operation switch 50 is provided in the orientation in which an indication such as a warning statement can be easily visible from the outside. Preferably, the display part 78 is provided in approximately the same direction as the operation surface 52 to which a force is applied by a finger when pushing the operation switch 50 in (see FIG. 4). This makes it easier for the user to see the information displayed on the display surface 82 before the push-in operation of the operation switch 50, and precautions regarding priming etc. can be confirmed by the user before the push-in operation of the operation switch 50 by the indication of the display surface 82. In this way, the display part 78 of the tab member 68 has an information display function for displaying priming-related information, and the display part 78 constitutes a display mechanism for displaying the information and providing the information to the user.

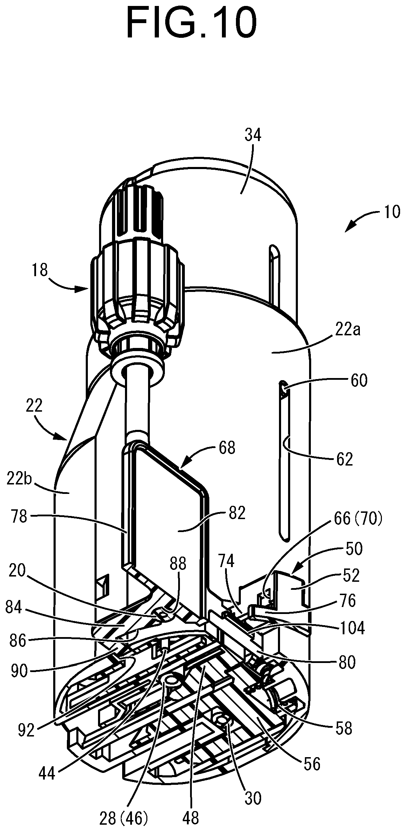

The insertion part 80 of the tab member 68 inserted in the tab insertion part 72 of the through hole 66 is allowed to be pulled out from the tab insertion part 72 by a predetermined amount. That is, as shown in FIG. 10, the tab member 68 can be pulled and moved in the third direction Z toward the display part 78 side until the side wall inner surface of of the concave groove 96 on the deep bottom part 100 side comes into contact with the opening edge of the tab insertion part 72 of the through hole 66. When the tab member 68 moves with respect to the operation switch 50 in the pull-out direction from the through hole 66 and the middle projection 74 of the operation switch 50 climbs over the convex part 102 of the tab member 68, the impact due to the climbing over is transmitted to the hands of the user. By so doing, the user can grasp that the tab member 68 has been pulled out to a predetermined position not only visually but also by a response.

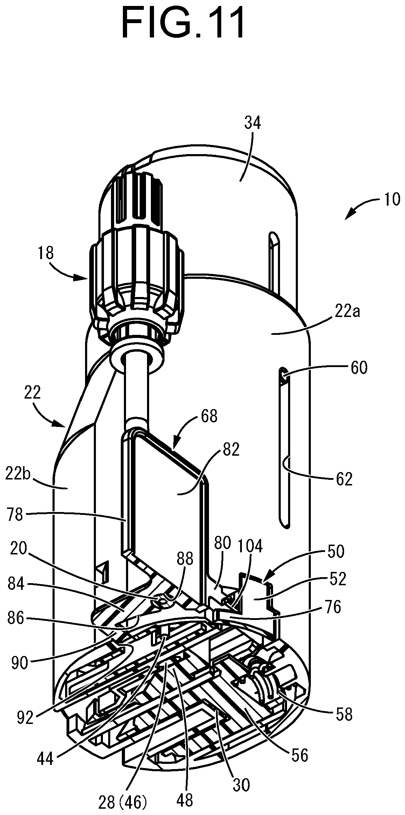

When the tab member 68 is pulled out to the above-mentioned position shown in FIG. 10, the pair of middle projections 74, 74 of the operation switch 50 are located on the extension of the deep bottom parts 100, 100 of the concave grooves 96, 96 in the tab member 68. By so doing, the action preventing mechanism by locking of the operation switch 50 and the tab member 68 in the second direction Y is released, and the operation switch 50 is allowed to displace relative to the tab member 68 in the second direction Y. Then, as shown in FIG. 11, the operation switch 50 is allowed to be pushed into the housing 22 in the second direction Y. Since the priming flow path 46 is closed by the operation switch 50 being pushed in, the release of the action preventing mechanism due to pulling-out of the tab member 68 is performed after the priming is completed. In the present practical embodiment, the action preventing mechanism is released by the pull-out operation of the tab member 68, but the action preventing mechanism may be released by another operation mode such as a push-in operation and a twisting operation.

As shown in FIG. 10, in the state before the operation switch 50 is pushed in, the pair of guide ribs 104, 104 of the insertion part 80 are inserted in advance between the pair of middle projections 74, 74 of the operation switch 50. With this configuration, when the pair of middle projections 74, 74 are inserted into the respective deep bottom parts 100 of the concave grooves 96, 96, for example, catching or the like between the edges of the pair of middle projections 74, 74 and the end face of the insertion part 80 is unlikely to occur, and the push-in operation of the operation switch 50 is smoothly realized.

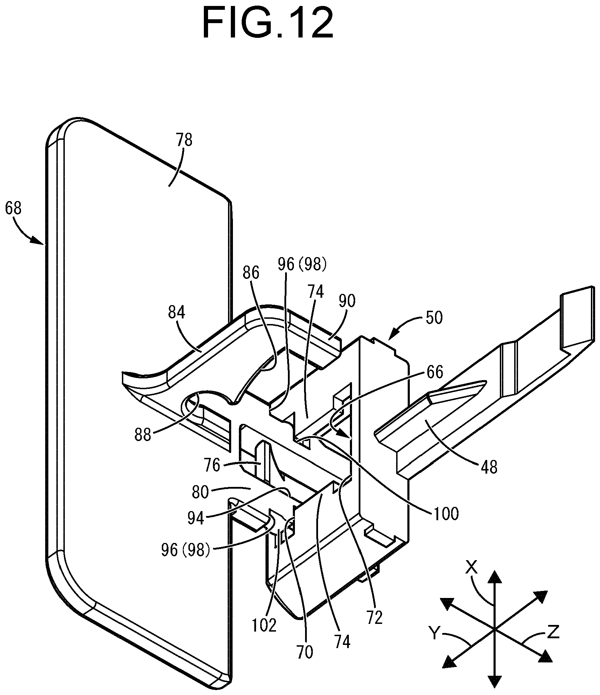

When the operation switch 50 is pushed in, the insertion part 80 of the tab member 68 is inserted through the removal part 70 of the through hole 66 as shown in FIGS. 11 and 12. Further, as shown in FIG. 12, the regulating protrusion 76 of the operation switch 50 is inserted in the lightening part 94 of the insertion part 80 inserted through the removal part 70, and the tab member 68 is prevented from being pushed into the through hole 66 by the tab member 68 being in contact and locked with the regulating protrusion 76.

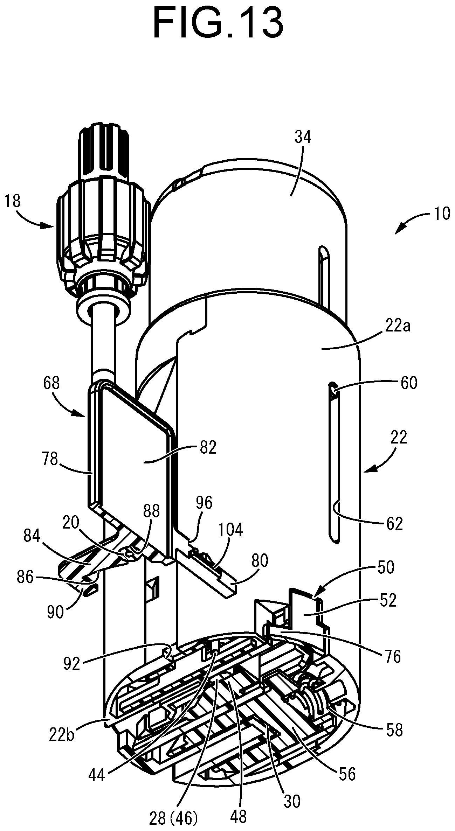

By the tab member 68 inserted through the removal part 70 being further pulled toward the display part 78 side in the third direction Z, the insertion part 80 is pulled out of the through hole 66, and the tab member 68 is separated from the operation switch 50, as shown in FIG. 13. By so doing, the tab member 68 is allowed to be removed from the drug solution injection controller 10 including the operation switch 50.

By detaching the tab member 68 from the operation switch 50 due to the operation of the operation switch 50 including the activation operation of the closing valve 48, the holding of the terminal connector 18 by the tab member 68 is allowed to be released. By so doing, the terminal connector 18 and the external flow path 20 can be moved away from the housing 22, thereby making it possible to easily connect the terminal connector 18 to the indwelling needle or the like on the patient side at a position away from the drug solution injection controller 10. In the present practical embodiment, the operation of the operation switch 50 includes an operation of removing and detaching the tab member 68 from the operation switch 50.

In the present practical embodiment, the tab member 68 is detached from the operation switch 50 and the connector holding part 84 is separated from the housing 22, so that the tube insertion part 88 is opened in a part in the circumferential direction. By so doing, the external flow path 20 and the terminal connector 18 can be detached from the connector holding part 84, thereby preventing the tab member 68 from interfering with the connection work when the terminal connector 18 is connected to the indwelling needle or the like on the patient side.

The tab member 68 can also be removed from the drug solution administration device 12 by being detached from the operation switch 50 of the drug solution injection controller 10. By the tab member 68 being removed from the drug solution administration device 12 after the operation of the operation switch 50, the display part 78 of the tab member 68 that displays information regarding priming can be removed from the drug solution injection controller 10, and hence the drug solution administration device 12. This makes it possible to prevent the patient or the like from being confused when he/she sees the indication on the display part 78 after the operation of the operation switch 50 is completed. That is, in the present practical embodiment, the display obstruction mechanism that obstructs the information display function of the display part 78 is constituted by the tab member 68 being detachable from the operation switch 50.

The display obstruction mechanism is not necessarily limited to that realized by removing the display part 78. For example, it would also be acceptable to constitute the display obstruction mechanism by the display part 78 being folded due to the operation of the operation switch 50 so that the display surface 82 is on the inside, thereby making it difficult to visually recognize the indication on the display surface 82 from the outside. Further, for example, it would also be possible to obstruct the information display function of the display part 78 by the display surface 82 being covered with a translucent or opaque cover attached to the display part 78. The display obstruction mechanism is preferably the one that loses the information display function of the display part 78, such as removing the display part 78 and covering the display surface 82 to be invisible. However, the display obstruction mechanism may alternatively be the one that deteriorates and obstructs the information display function such as making the display surface 82 difficult to be seen.

As described above, the tab member 68 attached to the operation switch 50 constitutes the action preventing mechanism for preventing erroneous pushing-in of the operation switch 50 before the completion of priming, the connection preventing mechanism for preventing connection of the terminal connector 18 to the indwelling needle or the like before the push-in operation of the operation switch 50, and the display mechanism for displaying the indication regarding the operation of the operation switch 50. In this way, since the tab member 68 has a plurality of functions, the operation of the tab member 68 due to the operation of the operation switch 50 performs all of the release of the action preventing mechanism, the release of the connection preventing mechanism, and the removal of the display part 78.

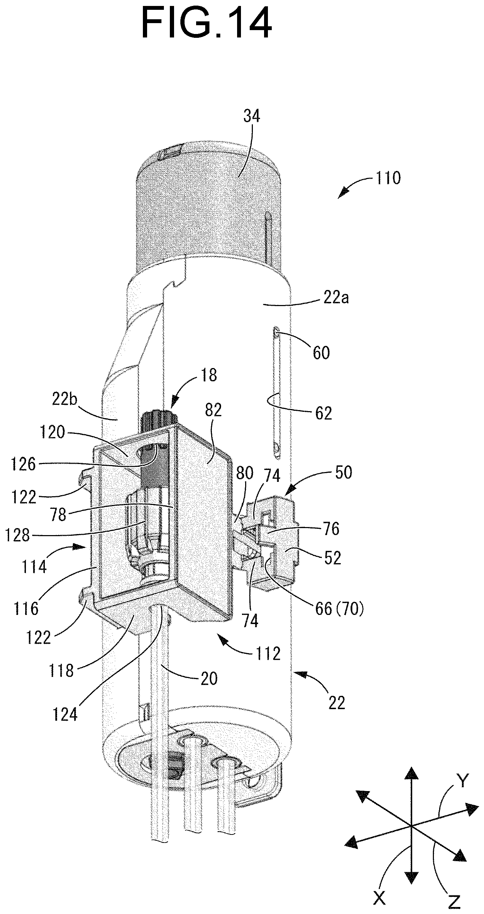

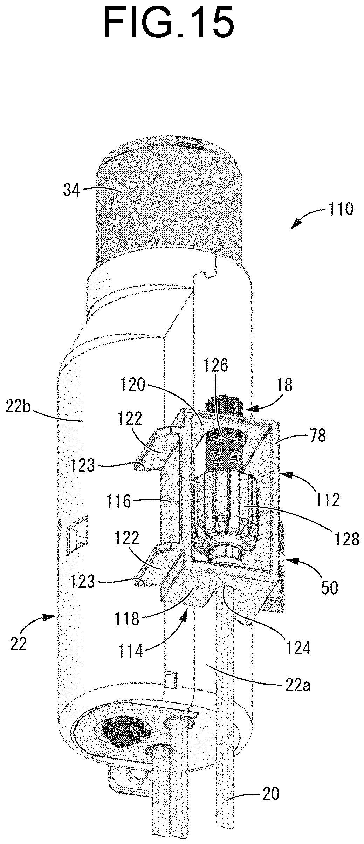

FIGS. 14 and 15 depict a drug solution injection controller 110 according to a second practical embodiment of the present invention. In the following description, components and parts that are substantially identical with those in the first practical embodiment will be assigned like symbols and not described in any detail.

The drug solution injection controller 110 includes a tab member 112. The tab member 112 is made of synthetic resin or the like. The tab member 112 has a structure in which a connector holding part 114 is integrally formed with the display part 78.

The connector holding part 114 integrally includes a rectangular plate-shaped support plate part 116 that extends approximately parallel to the display part 78, a distal end holding part 118 that connects the display part 78 and the support plate part 116 at the distal end, and a proximal end holding part 120 that connects the display part 78 and the support plate part 116 at the proximal end. Therefore, the connector holding part 114 has an approximately rectangular tube shape overall, and has high deformation rigidity. Meanwhile, the center axis of the connector holding part 114 is generally orthogonal to the center axis of the housing 22 (the first direction X).

In the support plate part 116, the distal end holding part 118 and the proximal end holding part 120 project from the surface opposed to the display part 78, while a pair of positioning parts 122, 122 are provided on the surface opposite to the display part 78. Each positioning part 122 projects from the support plate part 116 toward the side opposite to the display part 78, while extending toward the side where the connector holding part 114 is overlapped with the housing 22. The pair of positioning parts 122, 122 are supported by the housing 22 by being inserted into respective support holes 123, 123 penetrating the housing 22. With this configuration, for example, in the case where an external force acts on the connector holding part 114, the load can be further dispersed in comparison with the structure having only one positioning part 90 like the connector holding part 84 of the preceding practical embodiment. Therefore, it is possible to prevent the insertion part 80 from being broken.

The distal end holding part 118 has an approximately flat-plate shape that extends generally orthogonal to the center axis direction of the housing 22 (the first direction X). The distal end holding part 118 is configured such that, in the third direction Z, one end face has a curved shape which is overlapped with the outer peripheral surface of the housing 22, and a tube holding groove 124 is formed so as to open onto the one end face and extend in the third direction Z.

The proximal end holding part 120 has an approximately flat-plate shape that extends generally orthogonal to the center axis direction of the housing 22. The proximal end holding part 120 is configured such that, in the third direction Z, one end face has a curved shape which is overlapped with the outer peripheral surface of the housing 22, and a connector holding groove 126 is formed so as to open onto the one end face and extend in the third direction Z. In the present practical embodiment, the connector holding groove 126 is wider than the tube holding groove 124. However, the connector holding groove 126 and the tube holding groove 124 may have approximately the same groove width dimension as each other, or the connector holding groove 126 may be narrower than the tube holding groove 124.

The connector holding part 114 having such a structure is positioned with respect to the housing 22 by the support plate part 116, the distal end holding part 118, and the proximal end holding part 120 being overlapped on the housing 22 at the one end face in the third direction Z. Further, the insertion part 80 integrally formed with the display part 78 is inserted into the through hole 66 of the operation switch 50, and the pair of positioning parts 122, 122 are inserted into the support holes 123, 123 of the housing 22, so that the connector holding part 114 is attached to the housing 22 and the operation switch 50.

In such a state in which the connector holding part 114 is mounted on the housing 22, the tube constituting the external flow path 20 is inserted through the tube holding groove 124 of the distal end holding part 118. The terminal connector 18 provided at the terminal of the external flow path 20 has a diameter larger than the groove width of the tube holding groove 124 of the distal end holding part 118, and is located on the proximal end side of the distal end holding part 118. This configuration limits the amount of movement of the external flow path 20 toward the distal end side with respect to the distal end holding part 118.

Further, in such a state in which the connector holding part 114 is mounted on the housing 22, the terminal connector 18 provided at the terminal of the external flow path 20 is inserted through the connector holding groove 126 of the proximal end holding part 120. The terminal connector 18 is provided with a coupler 128 having a diameter larger than that of the portion inserted through the connector holding groove 126 on the distal end side of the proximal end holding part 120. This configuration limits the amount of movement of the terminal connector 18 toward the proximal end side with respect to the proximal end holding part 120.

Therefore, movement of the external flow path 20 and the terminal connector 18, which are held on the outer circumference of the housing 22 by the connector holding part 114, is limited in the length direction of the external flow path 20 on the opposite sides, and their positions with respect to the housing 22 are fixedly determined. Therefore, it is possible to more reliably prevent the terminal connector 18 held by the connector holding part 114 from being connected to the indwelling needle or the like. The connector holding part 114 of the present practical embodiment holds the terminal portion of the terminal connector 18 and the external flow path 20 side of the terminal connector 18 by the distal end holding part 118 and the proximal end holding part 120, respectively, thereby positioning the terminal connector 18 in a reliable manner.

The tab member 112 of the present practical embodiment can be pulled out of the operation switch 50 and the housing 22 due to operation of the operation switch 50, similarly to the tab member 68 of the first practical embodiment. Besides, by the step of pulling out the tab member 112 in the operation of the operation switch 50, as in the first practical embodiment, allowance of the activation of the closing mechanism (not shown) by operation of the operation switch 50, obstruction of the information display function of the display part 78 by removal of the tab member 112, and release of the terminal connector 18 held by the connector holding part 114 are realized.

While the present invention has been described in detail hereinabove in terms of the practical embodiments, the invention is not limited by the specific disclosures thereof. For example, the connector holding part 84 is not necessarily limited to the structure that holds the external flow path 20 in the vicinity of the terminal connector 18, and may have a structure that directly holds the terminal connector 18 by, for example, providing concave and convex portions that are fitted to the terminal connector 18. With this configuration, the terminal connector 18 will not move due to deformation of the external flow path 20, and the terminal connector 18 can be held in a more positioned state.

The connector holding part that holds the terminal connector 18 does not necessarily have to be detachably provided with respect to the housing 22. Specifically, for example, when the operation switch 50 is operated, the holding of the terminal connector 18 by the connector holding part may be released by allowing movement of the connector holding part such as tilting with respect to the housing 22.

In the preceding practical embodiment, one tab member 68 constitutes the connection preventing mechanism that prevents the terminal connector 18 from being connected before the operation switch 50 is operated, the action preventing mechanism that prevents the operation switch 50 from being operated before priming is completed, and the display mechanism that displays the indication regarding the operation of the operation switch 50. However, the connection preventing mechanism, the action preventing mechanism, and the display mechanism may be constituted by mutually different members. Further, the drug solution injection controller does not need to include all of the connection preventing mechanism, the action preventing mechanism, and the display mechanism, and it would be acceptable as long as at least one of them is included.

Moreover, the connection preventing mechanism, the action preventing mechanism, and the display mechanism do not need to be constituted by a member separate from the operation switch 50 such as the tab member 68, and may be constituted by a portion provided integrally with the operation switch 50. For example, if the display part 78 is integrally formed with the operation switch 50 and the portion connecting the operation switch 50 and the display part 78 is configured to be broken due to the operation of the operation switch 50, the display part 78 can be removed after the operation of the operation switch 50 is completed, as in the preceding practical embodiment. With this configuration, the display mechanism, which can be removed after the operation of the operation switch 50 is completed, can be constituted by the display part 78 provided integrally with the operation switch 50. Even in the case where the operation switch 50 and the display part 78 are separate members, it would also be possible to provide a friable part to the portion of the display part 78 connected to the operation switch 50, and to remove the display part 78 from the operation switch 50 by breaking the friable part.

### KEYS TO SYMBOLS

- 10: drug solution injection controller (first practical embodiment)
- 12: drug solution administration device
- 14: main line
- 16: main reservoir
- 18: terminal connector
- 20: external flow path
- 22: housing
- 22a: first half body
- 22b: second half body
- 24: sub-reservoir
- 26: sub-line
- 28: inflow line
- 30: outflow line
- 32: housing area
- 34: push button
- 36: coil spring
- 38: plunger
- 40: diaphragm part
- 42: base member
- 44: limiting flow path
- 46: priming flow path
- 48: closing valve
- 50: operation switch
- 52: operation surface
- 54: open-close valve
- 56: valve body
- 58: coil spring
- 60: mark part
- 62: slit
- 64: scale
- 65: three-way stopcock
- 66: through hole
- 68: tab member (holding member, action preventing member, display member)
- 70: removal part
- 72: tab insertion part
- 74: middle projection
- 76: regulating protrusion
- 78: display part
- 80: insertion part
- 82: display surface
- 84: connector holding part
- 86: concave end
- 88: tube insertion part
- 90: positioning part
- 92: positioning hole
- 94: lightening part
- 96: concave groove
- 98: shallow bottom part
- 100: deep bottom part
- 102: convex part
- 104: guide rib
- 110: drug solution injection controller (second practical embodiment)
- 112: tab member (holding member, action preventing member, display member)
- 114: connector holding part
- 116: support plate part
- 118: distal end supporting part
- 120: proximal end supporting part
- 122: positioning part
- 123: support hole
- 124: tube holding groove
- 126: connector holding groove
- 128: coupler

## Claims

1. A drug solution injection controller including a sub-reservoir connected via a sub-line to a main line for performing continuous administration of a drug solution such that rapid administration of the drug solution from the sub-reservoir by self-operation is enabled, the drug solution injection controller comprising:
a priming flow path configured to perform priming on at least one of the main line and the sub-line;
a closing mechanism configured to close the priming flow path; and
a connector holding part holding a terminal connector connected to the main line, wherein
holding of the terminal connector by the connector holding part is allowed to be released due to operation of an operation switch configured to activate the closing mechanism.

2. The drug solution injection controller according to claim 1, wherein the connector holding part is provided to a holding member separate from the operation switch, and holding of the terminal connector by the connector holding part is allowed to be released by operation of the holding member due to operation of the operation switch.

3. A drug solution injection controller including a sub-reservoir connected via a sub-line to a main line for performing continuous administration of a drug solution such that rapid administration of the drug solution from the sub-reservoir by self-operation is enabled, the drug solution injection controller comprising:
a priming flow path configured to perform priming on at least one of the main line and the sub-line;
a closing mechanism configured to close the priming flow path; and
an action preventing mechanism preventing operation of an operation switch configured to activate the closing mechanism, wherein
activation of the closing mechanism by operation of the operation switch is allowed due to release of the action preventing mechanism.

4. The drug solution injection controller according to claim 3, wherein the action preventing mechanism is constituted by an action preventing member separate from the operation switch being attached to the operation switch.

5. A drug solution injection controller including a sub-reservoir connected via a sub-line to a main line for performing continuous administration of a drug solution such that rapid administration of the drug solution from the sub-reservoir by self-operation is enabled, the drug solution injection controller comprising:
a priming flow path configured to perform priming on at least one of the main line and the sub-line;
a closing mechanism configured to close the priming flow path;
a display part having an information display function for priming-related information; and
a display obstruction mechanism configured to obstruct the information display function of the display part due to operation of an operation switch configured to activate the closing mechanism.

6. The drug solution injection controller according to claim 5, wherein the display part is provided to a display member separate from the operation switch and attached to the operation switch, and the display part is allowed to be removed by the display member being detached from the operation switch due to operation of the operation switch.

7. A drug solution injection controller including a sub-reservoir connected via a sub-line to a main line for performing continuous administration of a drug solution such that rapid administration of the drug solution from the sub-reservoir by self-operation is enabled, the drug solution injection controller comprising:
a priming flow path configured to perform priming on at least one of the main line and the sub-line;
a closing mechanism configured to close the priming flow path; and
a display part having an information display function for priming-related information including operation information of the closing mechanism.
